# FASCICULE DE BREVET EUROPEEN

(11) **EP 4 355 198 B1**
(45) Date de publication et mention de la délivrance du brevet: **29.07.2026**
(21) Numéro de dépôt: 22735217.6
(22) Date de dépôt: 13.06.2022
(51) Int. Cl.: A61B 5/00, A61B 5/16, G06F 3/01, G06F 3/0482, G06F 3/048

(54) **INTERFACE DE COMMUNICATION ADAPTEE EN FONCTION D'UNE EVALUATION COGNITIVE POUR PATIENTS PRIVES DE LA PAROLE**
KOMMUNIKATIONSSCHNITTSTELLE, DIE GEMÄSS EINER KOGNITIVEN BEURTEILUNG FÜR PATIENTEN MIT EINGESCHRÄNKTER SPRACHE ANGEPASST IST
COMMUNICATION INTERFACE ADAPTED ACCORDING TO A COGNITIVE EVALUATION FOR SPEECH-IMPAIRED PATIENTS

(30) Priorité: 14.06.2021 FR 2106239
(43) Date de publication de la demande: 24.04.2024
(73) Titulaire: Centre Hospitalier Régional Universitaire de Tours, 37000 Tours (FR)
(72) Inventeur: BODET-CONTENTIN, Laetitia, 37520 LA RICHE (FR); EHRMANN, Stephan, 37000 TOURS (FR)
(74) Mandataire: de Jong, Jean Jacques
(86) Numéro de dépôt international: PCT/FR2022/051120
(87) Numéro de publication internationale: WO 2022/263757

(56) Documents cités:
- CN-A- 111 443 665
- US-A1- 2010 092 929
- US-A1- 2013 239 015
- US-A1- 2017 344 209
- US-A1- 2018 330 801

## Description

La présente invention concerne un dispositif d'interface de communication adapté notamment aux patients hospitalisés en service de soins critiques (en réanimation, soins intensifs, surveillance continue).

En effet, les patients en unité de réanimation se trouvent majoritairement privés de la parole en raison de la présence d'une sonde d'intubation entre leurs cordes vocales ou d'une trachéotomie sans canule de phonation. Cependant, 50% d'entre eux se trouvent conscients et donc en état de communiquer. En outre, certains patients sont atteints d'une tétraparésie de réanimation entrainant une grande difficulté à se mobiliser suffisamment pour pouvoir utiliser des moyens de communication non verbaux comme l'écriture ou l'utilisation d'un clavier d'ordinateur. La position allongée ne facilite pas non plus l'usage d'un clavier.

La difficulté de communication peut également résulter de troubles neurologiques comme les syndromes confusionnels ou démentiels, le delirium de réanimation, les lésions neurologiques, ou les effets pharmacologiques ou secondaires de médicaments.

Pour les patients, cette incapacité à communiquer tout en étant conscient constitue une source de frustration, de déshumanisation, de dépersonnalisation, de perte d'estime de soi et de stress. Ainsi, la réanimation peut être vécue comme une torture, pouvant entrainer un syndrome de stress post-traumatique ou des séquelles psychologiques importantes à court et long terme.

Cette difficulté à communiquer place également les soignants en incapacité de comprendre les besoins et les demandes de leurs patients. Pour les proches des patients, elle conduit également à un sentiment d'impuissance.

Il existe donc un besoin de permettre à des patients n'ayant plus l'usage de la parole et des mains de pouvoir communiquer avec les soignants et leurs proches.

Il a été proposé d'utiliser un dispositif optique de suivi du regard pour commander un ordinateur. Ainsi, la demande de brevet US 2019/0272718 décrit un dispositif prévu pour être utilisé par un patient alité, intubé et incapable de bouger. Ce dispositif comprend un support configuré pour maintenir un écran d'affichage devant le visage du patient, un dispositif de suivi du regard et un dispositif de synthèse vocale. Le document US 2013/239015 A1 divulgue d'autres exemples de systèmes de communication entre un patient n'ayant plus l'usage de la parole et un soignant.

Cependant, ce dispositif s'avère fastidieux à utiliser par un patient particulièrement faible et épuisable rapidement. En outre, l'état d'un patient en service de réanimation peut s'améliorer ou à l'inverse se dégrader rapidement. D'une manière générale, les capacités de communication du patient peuvent être très variables d'un moment à un autre de sa prise en charge.

Dans ce contexte, il est souhaitable de proposer aux patients un moyen de communication qui soit efficace et adapté à leurs capacités cognitives et à leur capacité de concentration durant leur prise en charge.

L'invention est définie dans les revendications.

Des modes de réalisation concernent un procédé de communication, comprenant des étapes consistant à : équiper un patient d'un organe de commande et d'un écran d'affichage, exécuter une séquence de tests cognitifs utilisant l'écran d'affichage et l'organe de commande pour déterminer un score de compréhension du patient, sélectionner une interface de communication en fonction du score de compréhension, parmi plusieurs interfaces de communication ayant des complexités respectives distinctes, chaque interface de communication comprenant au moins une page d'accueil comportant des zones d'intérêt sélectionnables à l'aide de l'organe de commande, activer l'interface de communication sélectionnée et afficher à l'écran d'affichage la page d'accueil de l'interface de communication sélectionnée, détecter la désignation à l'aide de l'organe de commande d'une zone d'intérêt parmi les zones d'intérêt dans une page affichée à l'écran d'affichage, et émettre un message vocal à la suite de la détection de la désignation d'une zone d'intérêt.

Selon un mode de réalisation, les interfaces de communication comprennent : une première interface de communication comprenant uniquement une page d'accueil donnant accès à au plus dix commandes, et/ou une seconde interface de communication donnant accès à des commandes à partir de zones d'intérêt réparties sur la page d'accueil et plusieurs autres pages, les commandes de la seconde interface de communication étant activables en au plus six sélections à l'aide de l'organe de commande, et/ou une troisième interface de communication donnant accès à des commandes réparties sur un nombre illimité de pages.

Selon un mode de réalisation, chacun des tests cognitifs comprend des étapes consistant à : afficher une page comportant plusieurs images à l'écran d'affichage, émettre une consigne vocale en relation avec les images de la page affichée, acquérir une réponse du patient en relation avec la page affichée, à l'aide de l'organe de commande, et mettre à jour le score de compréhension en fonction de la réponse du patient, la réponse du patient étant un signal de sélection d'une zone de l'écran d'affichage ou des mouvements oculaires détectés à l'aide d'un dispositif d'oculométrie.

Selon un mode de réalisation, le procédé comprend l'incrémentation du score lorsque le patient a maintenu son regard sur l'une des images affichées, correspondant à la consigne vocale.

Selon un mode de réalisation, les tests cognitifs comprennent des tests de compréhension de mots, des tests de compréhension de phrases simples et des tests de compréhension de phrases complexes.

Selon un mode de réalisation, le score de compréhension comptabilise un nombre de réponses correctes du patient en relation avec les tests de compréhension de mots, un nombre de réponses correctes du patient en relation avec les tests de compréhension de phrases simples et un nombre total de réponses correctes du patient pour la séquence de tests cognitifs.

Selon un mode de réalisation, le procédé comprend, à la suite de l'exécution de la séquence de tests, des étapes consistant à : si le score de compréhension indique que le nombre total de réponses correctes est supérieur à une première valeur de seuil, sélectionner une première interface de communication donnant accès à des commandes réparties sur un nombre de pages illimité de pages, sinon si le score de compréhension indique que le nombre de réponses correctes en relation avec les tests de compréhension de phrases simples est supérieur à une seconde valeur de seuil, sélectionner une seconde interface de communication donnant accès à des commandes réparties sur pages et activables en au plus trois sélections à l'aide de l'organe de commande, et sinon si le score de compréhension indique que le nombre de réponses correctes en relation avec les tests de compréhension de mots simples est supérieur à une troisième valeur de seuil, sélectionner une troisième interface de communication donnant accès à au plus dix commandes, sinon se mettre en attente de l'exécution d'une nouvelle séquence de test.

Selon un mode de réalisation, le procédé comprend des étapes consistant à : sélectionner une séquence de tests cognitifs à exécuter pour déterminer un score de compréhension du patient, parmi plusieurs séquences de tests cognitifs, ou sélectionner un nombre de planches dans des groupes de planches de même niveau de difficulté pour constituer une séquence de tests cognitifs à exécuter pour déterminer un score de compréhension du patient.

Selon un mode de réalisation, la sélection d'une séquence de tests cognitifs à exécuter est effectuée en excluant une ou deux dernières séquences de tests cognitifs précédemment sélectionnées.

Selon un mode de réalisation, le procédé comprend des étapes consistant à : détecter au moyen d'un dispositif d'oculométrie que le patient a maintenu son regard sur une première zone d'intérêt dans une page affichée à l'écran pendant une durée supérieure à une valeur de seuil temporel, et émettre un message vocal correspondant à la première zone d'intérêt, ou afficher une nouvelle page en relation avec la première zone d'intérêt.

Selon un mode de réalisation, le procédé comprend des étapes consistant à : détecter par un dispositif d'oculométrie, une position de l'écran d'affichage fixée par le patient, afficher une icône à la position détectée, déterminer que la position détectée est fixe, lorsque la position détectée est fixe, animer l'icône pour indiquer un temps restant à fixer la position fixe, et activer une commande correspondant à la position fixe détectée, lorsque le temps écoulé depuis que la position détectée est fixe, dépasse une valeur de seuil de temps écoulé.

Selon un mode de réalisation, la valeur de seuil de temps écoulé est fixée en fonction de l'interface de communication sélectionnée, et/ou en fonction du score de compréhension obtenu lors de la dernière séquence de tests cognitifs exécutée.

Selon un mode de réalisation, la troisième interface de communication donne accès à un module de commande permettant de commander directement des dispositifs externes situés dans l'environnement proche du patient.

Des modes de réalisation peuvent également concerner un dispositif de communication comprenant un ordinateur, un écran d'affichage et un organe de commande, l'ordinateur étant configuré pour mettre en œuvre le procédé défini précédemment.

Selon un mode de réalisation, l'organe de commande comprend un dispositif d'oculométrie fournissant à l'ordinateur des positions successives du regard du patient sur l'écran, l'ordinateur étant configuré pour activer une commande associée à une zone d'intérêt affichée à l'écran, lorsque la position du regard du patient fournie par le dispositif d'oculométrie est maintenue dans la zone d'intérêt pendant une durée supérieure à une valeur de seuil temporel.

Des exemples de réalisation de l'invention seront décrits dans ce qui suit, à titre non limitatif en relation avec les figures jointes parmi lesquelles :
[Fig. 1] la f igure 1 représente schématiquement un mode de réalisation d'un dispositif d'aide à la communication adapté à des patients alités et privés de la parole,
[Fig. 2] la f igure 2 représente schématiquement une architecture matérielle du dispositif d'aide à la communication, selon un mode de réalisation,

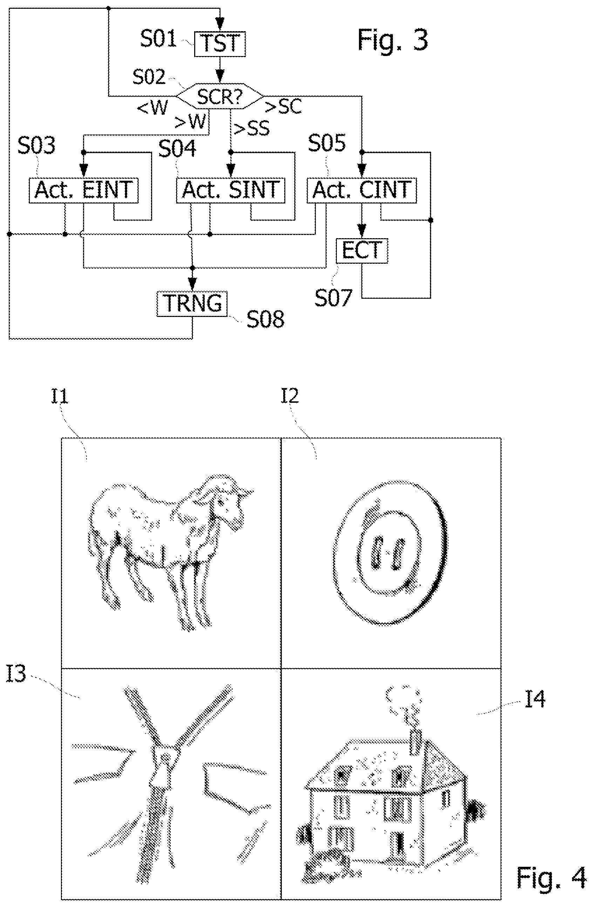

[Fig. 3] la f igure 3 représente schématiquement une architecture fonctionnelle du dispositif d'aide à la communication, selon un mode de réalisation,

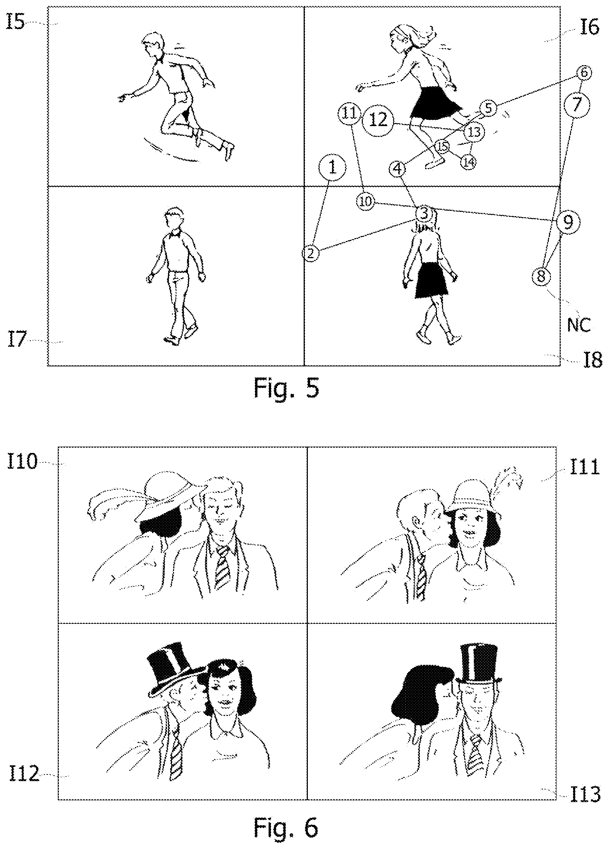

[Fig. 4] [Fig. 5] [Fig. 6] les figures 4 à 6 représentent des exemples de pages affichées à l'écran durant un test cognitif, selon un mode de réalisation,
[Fig. 7] [Fig. 8] [Fig. 9] [Fig. 10] [Fig. 11] [Fig. 12] [Fig. 13] [Fig. 14] [Fig. 15] les figures 7 à 15 représentent des exemples de pages affichées par le dispositif d'aide à la communication, selon un mode de réalisation,

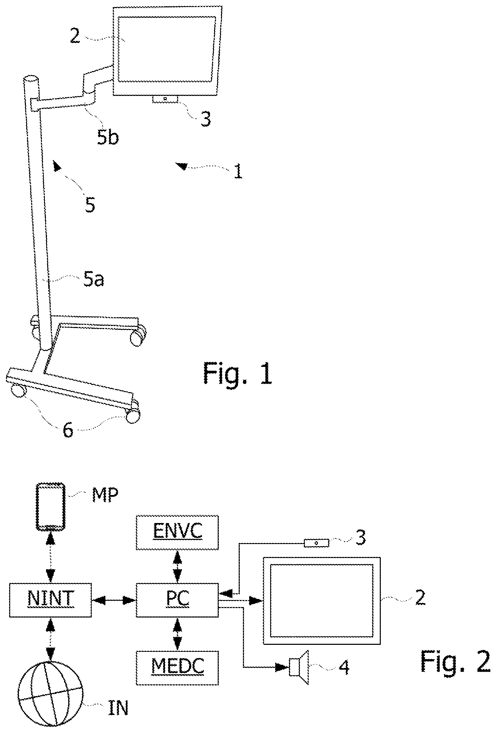

La f igure 1 représente un dispositif 1 d'aide à la communication. Selon un mode de réalisation, le dispositif 1 est adapté à des patients alités et privés de la parole. La f igure 2 représente l'architecture matérielle du dispositif 1. Sur les figures 1 et 2, le dispositif 1 comprend un ordinateur PC connecté à un écran d'affichage 2, un haut-parleur 4, un organe de commande 3 tel qu'un dispositif d'oculométrie ou de suivi du regard ("eye-tracking"), et un circuit de communication NINT. L'écran 2 est fixé sur un support 5 réglable, configuré pour permettre de disposer et orienter l'écran 2 à une certaine distance devant le visage du patient quelle que soit la position, allongée ou assise, de ce dernier. Le support 5 peut comprendre un axe vertical 5a, un bras articulé 5b fixé à l'axe vertical de manière à être réglable en hauteur, et un organe de fixation (non représenté) pour fixer l'écran 2 à l'extrémité du bras 5b. L'organe de fixation peut comporter une rotule pour faciliter l'orientation de l'écran 2 face au visage du patient. Le support 5 peut être monté sur des roulettes 6. L'ordinateur PC peut être un ordinateur individuel ou une tablette, par exemple une tablette tactile.

Le dispositif d'oculométrie 3 peut être associé à l'écran 2et comprendre un ou plusieurs capteurs d'image (par exemple en infrarouge ou en lumière visible) connectés à un dispositif d'analyse d'images configuré pour détecter les yeux d'un utilisateur et déterminer une direction du regard, et éventuellement d'autres phénomènes comme un clignement de paupières. Le dispositif d'oculométrie 3 peut également se présenter sous la forme de lunettes à porter par le patient, ou de toute autre dispositif ayant la fonction de détecter position d'un point observé par une personne. Le dispositif d'analyse d'images peut également être configuré pour détecter d'autres phénomènes pouvant être utilisés pour déterminer un état de vigilance ou plus généralement les capacités de communication du patient.

Le circuit de communication NINT peut comprendre une carte réseau notamment de type Ethernet, WiFi, Bluetooth, etc., pour se connecter par exemple au réseau Internet IN et/ou à un téléphone mobile MP.

L'ordinateur PC peut également être connecté à des appareils médicaux MEDC et à des dispositifs externes ENVC. Les appareils médicaux peuvent comprendre des appareils de mesure de la fréquence cardiaque (électrocardiogramme), de la fréquence respiratoire de la saturation en oxygène ou des ondes cérébrales (électroencéphalogramme). Les dispositifs externes ENVC peuvent comprendre un poste de télévision, un poste de radio, une télécommande de volet pour obturer une fenêtre de la chambre où se trouve le patient, une télécommande d'inclinaison du lit du patient, une télécommande d'une climatisation de la chambre, une télécommande pour appeler un soignant. L'ordinateur PC peut également être connecté à un capteur d'image pour recevoir des images du visage du patient ou de l'ensemble du patient et être configuré pour effectuer une analyse des images du visage du patient afin d'évaluer la douleur ressentie, et/ou pour évaluer la capacité du patient à utiliser sa main pour utiliser un crayon ou un clavier d'ordinateur.

D'autres organes de commande peuvent être connectés à l'ordinateur PC comme une souris, un clavier ou un écran tactile, pour permettre à un soignant de commander l'ordinateur. Un bouton poussoir peut également être connecté à l'ordinateur PC pour permettre au patient de valider une sélection ou déclencher une alarme, s'il en a la capacité.

La f igure 3 représente un exemple d'architecture fonctionnelle d'un programme exécuté par l'ordinateur PC, selon un mode de réalisation. Le programme comprend un module de test TST, plusieurs modules d'interface de communication EINT, SINT, CINT, un module d'interface de commande d'équipements externes ECT, et un module d'entrainement cognitif TRNG.

Les modules d'interface de communication peuvent comprendre un module d'interface élémentaire EINT, un module d'interface simple SINT, et un module d'interface complexe CINT.

A l'activation du programme, l'ordinateur PC exécute le module de test TST à l'étape S01. Le module de test TST déroule une séquence de tests des capacités cognitives et de communication du patient et calcule un score SCR en fonction des réponses fournies par le patient. Selon un mode de réalisation, la séquence de tests cognitifs est adaptée à l'état du patient (en soins critiques) et à l'utilisation d'un dispositif de suivi du regard. Les tests déroulés par le module de test TST sont configurés pour être réalisables quelle que soit la position du patient (allongé, assis), et à plusieurs reprises pendant l'hospitalisation du patient. La durée des tests est donc choisie suffisamment courte, par exemple inférieure à 5 minutes. A l'issue d'une session de test, le module de test peut afficher et/ou annoncer vocalement le résultat du test, l'archiver et l'envoyer à un ordinateur distant.

A tout instant, un soignant peut évaluer les capacités physiques du patient pour déterminer l'organe de commande le mieux adapté. Ainsi, si le patient n'a pas l'usage des doigts d'une main, le dispositif d'oculométrie seul apparait le mieux adapté. Si le patient peut presser un bouton poussoir, ce dispositif peut être associé au dispositif d'oculométrie pour valider une zone d'intérêt observée. Si le patient peut bouger un bras et une main, il peut utiliser un clavier ou un pavé tactile indépendant, ou bien un écran tactile placé sur l'écran d'affichage 2.

A l'étape S02, le score SCR est comparé à des valeurs de seuil pour activer une interface de communication sélectionnée parmi plusieurs interfaces de communication prédéfinies en fonction du score. Ainsi, par exemple, si le score SCR est inférieur à une valeur de seuil W, l'ordinateur PC n'active aucune interface de communication. Si le score SCR est supérieur à la valeur de W et inférieur à une valeur de seuil SS, l'ordinateur PC active le module d'interface élémentaire EINT, à l'étape S03. Si le score SCR est supérieur à la valeur de seuil SS, mais inférieur à une valeur de SC, l'ordinateur PC active le module d'interface simple SINT, à l'étape S04. Si le score SCR est supérieur à la valeur de SC, l'ordinateur PC active le module d'interface complexe CINT, à l'étape S05. Ainsi, le patient a accès à une interface de communication dont la complexité est sélectionnée en fonction d'une évaluation de ses capacités cognitives et de communication.

Chacun des modules d'interface EINT, SINT, CINT est configuré pour présenter des icônes de commande associées à une commande et éventuellement à une information textuelle indiquant la signification de l'icône, et pour prendre en compte et afficher le parcours oculaire du patient sur l'écran 2, c'est-à-dire les positions successives d'un point de l'écran 2 observé par le patient.

Selon un mode de réalisation, la position du point de l'écran 2 fixé par le patient est indiquée par un pointeur (M1 sur la f igure 7). Lorsque ce point est fixe, le pointeur M1 peut être animé pour indiquer le temps restant pendant lequel le patient doit fixer son regard pour sélectionner une icône affichée et ainsi activer la commande correspondante. Le temps de fixation d'une icône pour activer la commande correspondante peut être réglable en fonction de l'état du patient, par exemple entre 1 et 2,5 s, ou dépendre de l'interface de communication activée. Selon un exemple de réalisation, le temps de fixation d'une icône peut être maximum (par exemple 2,5 s) lorsque l'interface de communication EINT est sélectionnée, minimum (par exemple 1 s) lorsque l'interface de communication CINT est sélectionnée, et fixée à une valeur intermédiaire, par exemple entre 1,5 et 2 s lorsque l'interface de communication SINT est sélectionnée.

Les modules d'interface EINT, SINT, CINT diffèrent les uns des autres par les commandes auxquelles ils donnent accès, par le nombre d'icônes de commande affichées simultanément, ainsi que par le nombre maximum de sélections à effectuer pour accéder à une commande.

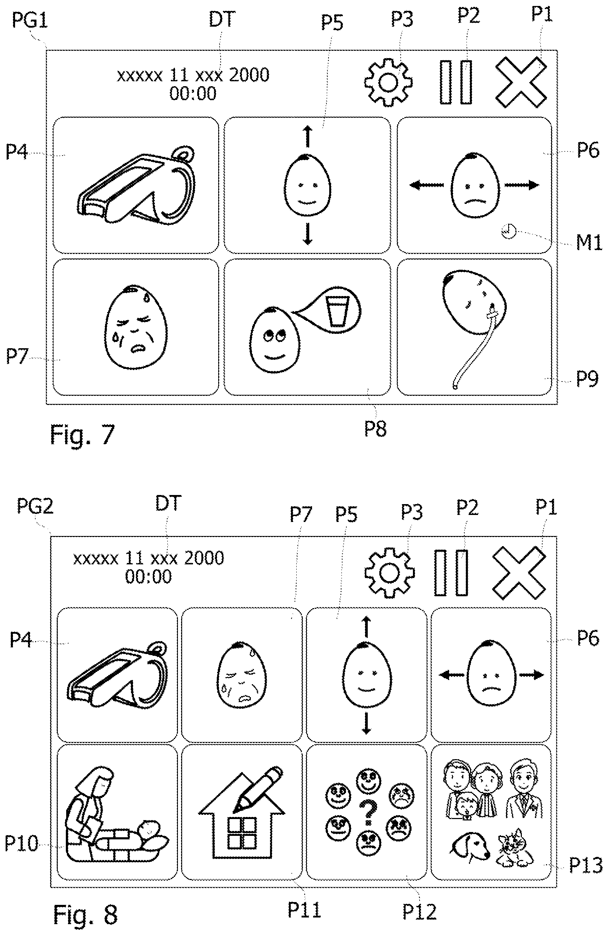

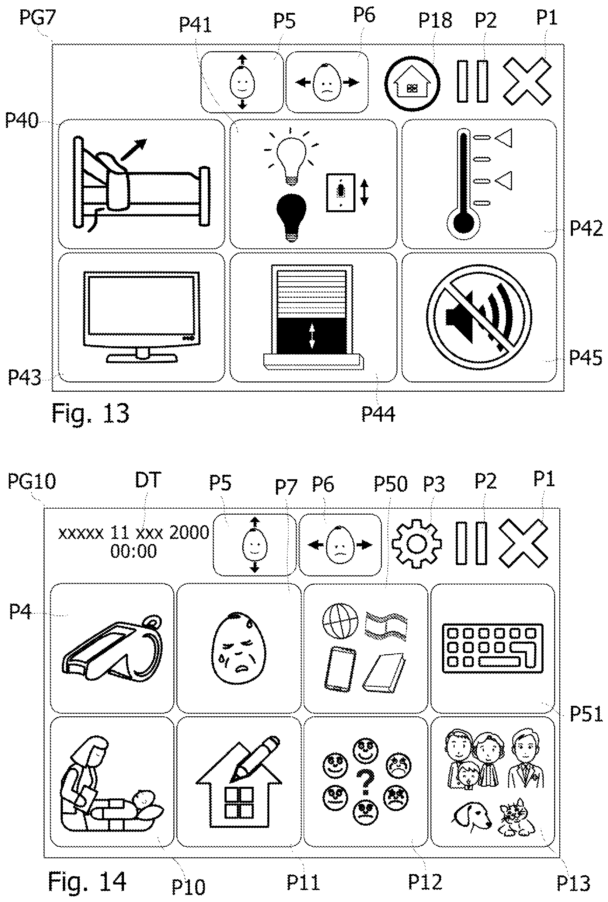

Les modules d'interface EINT, SINT et CINT affichent une page d'accueil respective PG1, PG2, PG10 (figures 7, 8 et 14) présentant des icônes de commande donnant accès à différentes commandes permettant au patient de communiquer avec son entourage. Chaque icone de commande peut être située dans une zone d'intérêt où le patient doit fixer son regard pour activer la commande correspondante. Les icônes présentées peuvent être animées, notamment pour améliorer leur intelligibilité afin de déterminer la commande qui leur est attribuée.

Chaque page d'accueil PG1, PG2, PG10 affichée par les modules d'interface EINT, SINT et CINT peut indiquer la date, l'heure et le lieu, afin de permettre au patient de se situer dans le temps et l'espace.

Parmi les icônes de commande présentées dans la page d'accueil respective des modules d'interface EINT, SINT et CINT, il peut être prévu une icône permettant d'activer le module d'entrainement cognitif TRNG, à l'étape S08. Le module TRNG peut également être activé à la fin d'une séquence de tests cognitifs exécutée par le module de test TST. A la fin d'une phase d'entrainement exécutée par le module TRNG, ou à partir des pages d'écran d'accueil affichées par les modules d'interface EINT, SINT et CINT, le patient peut également activer le module de test TST pour réaliser un nouveau test cognitif (activer le module de test TST).

Le module d'entrainement cognitif TRNG peut proposer au patient un programme de réadaptation ou de rééducation, qui peut être défini en fonction du dernier score SCR obtenu à l'issue d'une séquence de tests déroulée par le module de test TST. Le module TRNG peut utiliser la réalité virtuelle. Ainsi, à chaque session du programme de rééducation, le module TRNG peut placer le patient dans un environnement lui permettant de réaliser des actes de la vie quotidienne simples et/ou de complexité croissante, notamment afin de diminuer les perturbations du cycle veille/sommeil. Les actes de la vie quotidienne sont par exemple aller chercher son pain, aller chez le coiffeur, emmener ses enfants à l'école. Le programme de rééducation est adapté aux caractéristiques du patient (âge, sexe, statut socio-professionnel, entourage familial, ...), du score obtenu à la dernière séquence de tests déroulée par le module de test TST et de la progression de ces résultats. La durée de chaque session du programme de rééducation est adaptée à la capacité d'attention et de l'état de faiblesse du patient. Cette durée peut être déterminée au choix du patient ou en fonction du dernier score SCR déterminé par le module de test TST.

Le module d'interface CINT donne accès à un module de commande ECT (étape S07) permettant de commander directement des dispositifs externes situés dans l'environnement proche du patient, par exemple à l'aide d'une liaison infrarouge ou radio. Ces dispositifs externes peuvent comprendre par exemple un poste de télévision, un poste de radio, un régulateur de température ambiante, une télécommande de volets de fenêtre, une télécommande de l'intensité de l'éclairage ambiant, une télécommande d'inclinaison d'un lit ou d'un fauteuil, et d'autres interactions avec l'environnement, l'équipe de soins et des appareils biomédicaux tels qu'une pompe à morphine.

Selon un mode de réalisation, un module de calibration peut être activé à l'initialisation du dispositif de communication, avant d'activer le module de test TST. Le module de calibration est configuré pour contrôler la luminosité de l'image du visage du patient et ajuster la distance entre le visage du patient et l'écran d'affichage 2. L'étape de calibration peut être suivie d'une sélection d'un organe de commande adapté à l'état du patient. Cet organe de commande peut être le dispositif de suivi du regard 3 seul ou en combinaison avec un bouton poussoir placé dans la main du patient, et à utiliser pour valider une sélection effectuée à l'aide du dispositif 3. Si le patient possède le plein usage d'un bras et d'une main, l'organe de commande peut être un clavier, un pavé tactile ou un écran tactile placé sur l'écran d'affichage 2.

Selon un mode de réalisation, le module de test TST est configuré pour afficher successivement à l'écran 2 des planches d'une série de planches divisées en zones d'intérêt, par exemple de 2 à 4 zones d'intérêt. Lors de l'affichage d'une planche, une consigne vocale désignant l'une des zones d'intérêt est diffusée par le haut-parleur 4. La consigne vocale peut être un enregistrement d'une voix humaine réelle. Le patient doit placer son regard sur la zone d'intérêt désignée par la consigne vocale. Lorsqu'un temps d'affichage de planche est écoulé, une nouvelle planche est affichée et une nouvelle consigne vocale est émise. Au fur et à mesure de l'affichage des planches, le module de test TST capte les réponses fournies par le patient, et calcule un score SCR en l'incrémentant en fonction de ces réponses.

La série de planches de test associées chacune à une consigne, a pour but de tester les capacités cognitives du patient telles que la capacité de compréhension orale et de communication et la capacité du patient à fixer son regard sur une zone limitée de l'écran 2.

Selon un mode de réalisation, chaque planche de la série de planches affichées successivement est associée à un niveau de difficulté. Les planches dans la série de planches peuvent être ordonnées par niveau de difficulté croissant ou présentées dans un ordre aléatoire. L'ordre de présentation des planches peut également être défini au fur et à mesure en fonction des réponses déjà fournies par le patient durant le test. Par exemple, la série de planches comporte trois niveaux de difficulté pour évaluer la capacité de compréhension orale du patient, à savoir un premier niveau de compréhension de mots, un second niveau de compréhension de phrases simples, et un troisième niveau de compréhension de phrases plus complexes.

La f igure 4 représente un exemple de planche du premier niveau de difficulté, affichée par le module de test TST durant l'étape S01. Cette planche comporte quatre zones d'intérêt I1-I4 présentant chacune une image, à savoir l'image d'un mouton I1, d'un bouton I2, d'une fermeture à glissière I3 et d'une maison I4. La consigne vocale diffusée lors de l'affichage de la planche est par exemple "regardez le plus longtemps possible le bouton" ou simplement "bouton".

Selon un mode de réalisation, les consignes vocales du second niveau de difficulté comprennent des phrases comportant uniquement un sujet et un verbe, comme par exemple "la fille marche", "l'homme mange", "la femme boit". Les consignes vocales du second niveau de difficulté peuvent également comprendre des phrases comportant un sujet, un verbe à la forme active et un objet comme par exemple "le garçon suit le chien", "le cheval tire le garçon", "la femme suit le chien et la voiture". La f igure 5 représente un exemple de planche du second niveau de difficulté. Cette planche rassemble quatre images I5-I8, à savoir l'image d'un garçon qui court I5, d'une fille qui court I6, d'un garçon qui marche I7 et d'une fille qui marche I8. La consigne vocale diffusée lors de l'affichage de la planche est par exemple "regardez le plus longtemps possible la fille qui court".

Selon un mode de réalisation, les consignes vocales du troisième niveau de difficulté comprennent des phrases comportant une localisation d'objet comme par exemple "le chat est derrière la chaise", un verbe à la forme passive, comme par exemple "le chien est poussé par le garçon", et des phrases comportant une proposition subordonnée comme par exemple "c'est le garçon qui regarde le chien", "l'homme qui porte un chapeau embrasse la femme". La f igure 6 représente un exemple de planche du troisième niveau de difficulté. Cette planche comporte quatre images I10-I13, à savoir l'image I10 d'une femme portant un chapeau et embrassant un homme, l'image I11 d'un homme embrassant une femme portant un chapeau, l'image I12 d'un homme portant un chapeau et embrassant une femme, et l'image I13 d'une femme embrassant un homme portant un chapeau. La consigne vocale diffusée lors de l'affichage de la planche est par exemple "regardez le plus longtemps possible l'homme qui porte un chapeau embrasse la femme".

Selon un mode de réalisation, le module de test TST est configuré pour afficher chaque planche pendant une certaine durée, par exemple 6 secondes, et enregistrer le parcours oculaire du patient en relation avec chaque planche affichée.

Selon un mode de réalisation, l'ordinateur PC est configuré pour analyser les parcours oculaires enregistrés pour déterminer le score SCR. L'enregistrement du parcours oculaire peut comporter des disques colorés, numérotés par ordre chronologique et dont la taille correspond au temps de fixation du regard du patient sur la position du centre du disque. La couleur de chaque disque indique si le disque se trouve sur l'image correspondant à la bonne réponse ou non. Par exemple, les disques sur l'image correspondant à la bonne réponse sont verts et les disques se trouvant sur les autres images sont rouges. Dans l'exemple de la f igure 5, le parcourt oculaire comprend 15 positions NC numérotées de 1 à 15. Les positions 1, 4, 5 et 11 à 15 se trouvent dans zone d'intérêt correspondant à a bonne réponse.

L'analyse du parcours oculaire peut comprendre le calcul du temps pendant lequel le patient a maintenu son regard sur la zone d'intérêt correspondant à la consigne. Ainsi, il peut être considéré que le patient a fourni une bonne réponse s'il a maintenu son regard sur la zone d'intérêt correspondant à la consigne vocale pendant un temps supérieur à une valeur de seuil. Cette valeur de seuil est par exemple fixée à la moitié du temps d'affichage de la planche.

Le module de test TST peut également être configuré pour distinguer les mauvaises réponses et les absences de réponse. Une mauvaise réponse se produit lorsque le patient a sélectionné ou maintenu son regard sur une zone d'intérêt ne correspondant pas à la consigne vocale pendant un temps supérieur à la valeur de seuil. Une absence de réponse se produit lorsque le patient n'a pas sélectionné de zone d'intérêt pendant le temps imparti ou a regardé la planche affichée pendant un temps inférieur à la valeur de seuil.

Le module de test TST est configuré pour comptabiliser les bonnes réponses en fonction du niveau de difficulté associé à chaque planche. Le module de test TST peut également être configuré pour comptabiliser les mauvaises réponses en fonction du niveau de difficulté associé à chaque planche. Le module de test TST peut également être configuré pour comptabiliser les absences de réponse.

Selon un mode de réalisation, le module de test TST est configuré pour sélectionner une série de planches à afficher successivement parmi plusieurs séries alternatives mémorisées par l'ordinateurs PC, afin de permettre au patient de réaliser le test plusieurs fois durant son séjour tout en évitant un phénomène d'apprentissage. La série de planches à afficher peut être sélectionnée aléatoirement parmi les séries de planches disponibles, en excluant la dernière ou les deux dernières séries de planches sélectionnées.

Selon un mode de réalisation, chacune des planches de la série de planches est associée à plusieurs consignes vocales, l'une des consignes vocales associées étant sélectionnée aléatoirement lors de l'affichage de la planche.

Selon un autre mode de réalisation, le module de test TST est configuré pour constituer dynamiquement la série de planches à afficher successivement, en sélectionnant aléatoirement un certain nombre de planches dans des groupes de planches de même niveau de difficulté. Les planches sélectionnées lors du dernier ou des deux derniers tests exécutés peuvent être exclues de la sélection.

A l'issue d'un test, le score SCR peut révéler quatre situations possibles, à savoir :
- Cas 1, le patient comprend les phrases complexes, par exemple lorsqu'il a fourni moins de trois réponses erronées pour l'ensemble des planches de la séquence de test,
- Cas 2, le patient comprend les phrases simples par exemple lorsqu'il a fourni moins de deux réponses erronées pour les planches du second niveau de compréhension de phrases simples,
- Cas 3, le patient comprend des mots, par exemple lorsqu'il a fourni une réponse correcte pour chacune des planches du premier niveau de compréhension de mot,
- Cas 4, le patient n'est pas en état d'utiliser le dispositif de communication, par exemple lorsqu'il a fourni au moins une réponse erronée pour les planches du premier niveau ou n'a pas regardé les planches.

Dans le cas 1, le module d'interface complexe CINT est activé. Dans le cas 2, le module d'interface simple SINT est activé. Dans le cas 3, le module d'interface élémentaire EINT est activé. Dans le cas 4, aucun des modules d'interface EINT, SINT, CINT n'est activé, le patient étant considéré incapable d'utiliser le dispositif de communication.

Selon un mode de réalisation, le module d'interface élémentaire EINT est configuré pour afficher une unique page de commande donnant accès à un nombre limité de commandes permettant au patient de communiquer sur des besoins élémentaires.

Selon un mode de réalisation, le module d'interface intermédiaire SINT est configuré pour afficher une première page de commande donnant accès à un nombre plus étendu de commandes pouvant permettre d'accéder à trois autres pages de commande successives maximum, de sorte que toutes les commandes accessibles puissent être activées en six sélections au maximum depuis la page d'accueil.

Selon un mode de réalisation, le module d'interface complexe CINT complète les commandes accessibles par le module d'interface simple SINT en donnant accès à un clavier, à Internet et au téléphone du patient. Le module d'interface complexe CINT peut également donner un accès direct au module de commande ECT.

Selon un mode de réalisation, l'ordinateur PC est configuré pour analyser les parcours oculaires enregistrés et déterminer une durée de fixation d'une icône affichée pour déclencher la commande associée à l'icône.

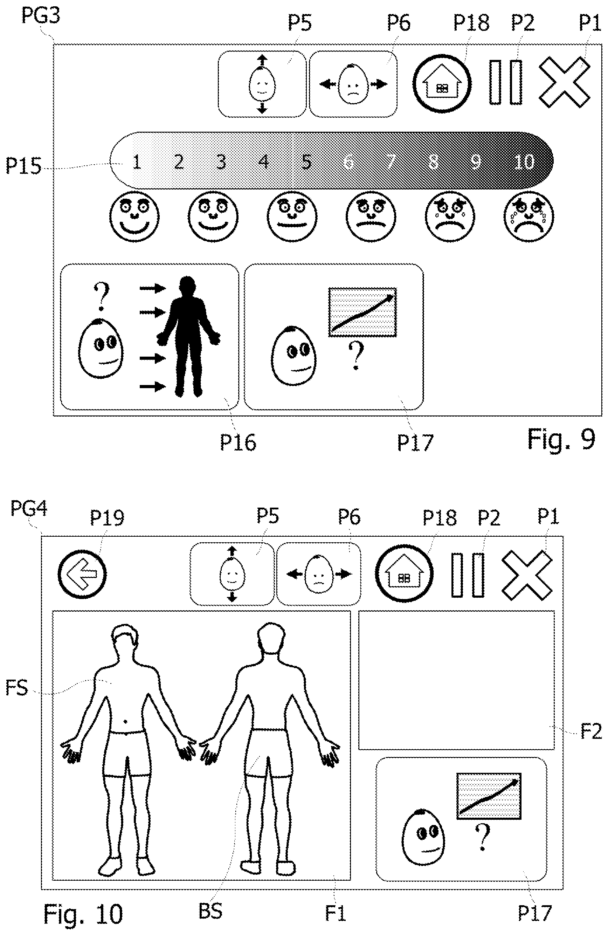

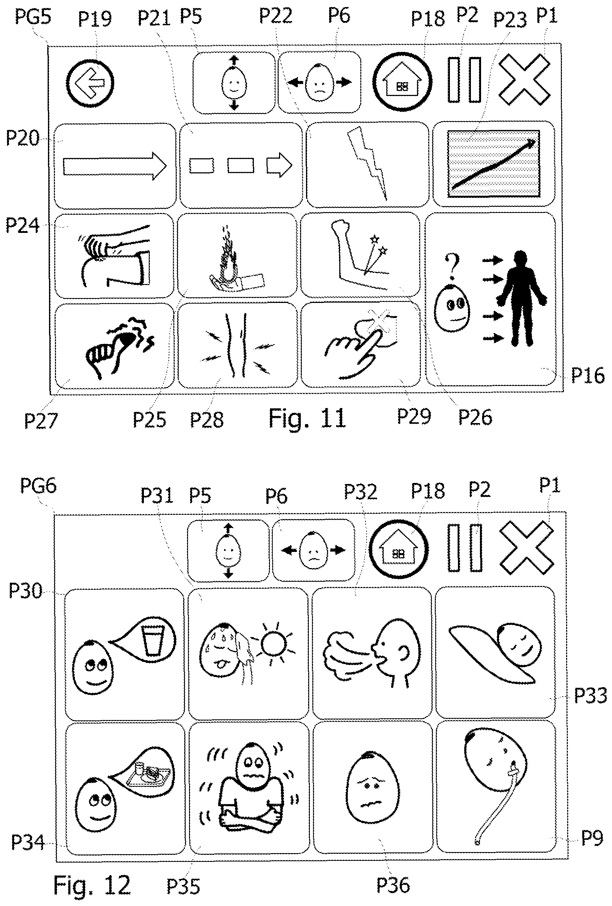

Les figures 7 à 15 représentent différentes pages PG1-PG8, PG10, PG11 affichées par l'un ou l'autre des modules d'interface de communication. Ces pages comportent généralement un bandeau supérieur et des zones d'intérêt comportant chacune un pictogramme ou une icône, qui peut être associé à une information textuelle indiquant la commande associée au pictogramme.

La f igure 7 représente un exemple de page d'accueil PG1 affichée par le module d'interface élémentaire SINT. La page d'accueil PG1 comprend un bandeau supérieur et six zones d'intérêt P4, P5, P6, P7, P8. Le bandeau supérieur affiche la date et l'heure courante DT, et comporte trois pictogrammes P1, P2, P3 associés respectivement à des commandes de sortie P1 de l'application de communication, de mise en pause P2 du dispositif d'oculométrie 3 et d'accès P3 à d'autres fonctionnalités du dispositif de communication. Ces autres fonctionnalités peuvent comprendre l'activation des modules de test TST et d'entrainement cognitif TRNG, l'activation d'un module de calibration du dispositif d'oculométrie 3, et la définition de paramètres de fonctionnement du dispositif de communication. La zone d'intérêt P4 est associée au déclenchement d'une alarme pour permettre au patient de demander une intervention urgente d'un soignant. Cette alarme peut être émise sous forme sonore et/ou transmise au système d'alarme existant de la chambre et/ou transmise à un ordinateur distant.

Les zones d'intérêt P5, P6 permettent au patient de répondre "OUI" et "NON", respectivement, à une question qui lui est posée oralement par une personne présente à côté de lui. La zone d'intérêt P7 permet au patient de signaler qu'il ressent une douleur. La zone d'intérêt P8 permet au patient de signaler qu'il a soif. La zone d'intérêt P9 permet au patient de signaler que le tuyau d'intubation le gêne. A la suite de la sélection de l'une des zones d'intérêt P4-P9, le dispositif de communication peut commander la diffusion orale du texte correspondant à la zone d'intérêt.

La f igure 8 représente un exemple de page d'accueil PG2 affichée par le module d'interface simple SINT. La page d'accueil PG2 comprend un bandeau supérieur et huit zones d'intérêt dont les zones d'intérêt P4, P7, P5 et P6 de la f igure 7, et des zones d'intérêt P10, P11, P12 et P13. Chaque zone d'intérêt P10-P13 comporte également un pictogramme qui peut être associé à une information textuelle indiquant la commande associée au pictogramme. Le bandeau supérieur peut être identique à celui de la page PG1.

Chacune des zones d'intérêt P7 et P10 à P13 donne accès à une page de commande rassemblant des questions courantes sélectionnables, posées par les patients en soin critique. La zone d'intérêt P7 donne accès à une page de communication permettant au patient de préciser comment il perçoit la douleur. La zone d'intérêt P10 donne accès à une page de communication permettant au patient de demander un soin particulier, tel qu'une toilette, un massage, la diffusion de parfum ou de musique. La zone d'intérêt P11 donne accès à une page de communication permettant au patient de demander une action sur son environnement proche, à savoir par exemple sur son lit, sur la lumière, le bruit ou la température ambiante. La zone d'intérêt P12 donne accès à une page de communication permettant au patient de signaler un inconfort, par exemple relatif à la soif, la faim, la chaleur, son sommeil, sa respiration, son humeur, ou encore relatif au tuyau d'intubation. La zone d'intérêt P13 donne accès à une page de communication permettant au patient de sélectionner une personne parmi ses proches ou un soignant avec qui il souhaite communiquer.

La f igure 9 représente un exemple de page de communication PG3 sur une douleur ressentie, accessible à partir de la zone d'intérêt P7 (f igure 8). La page PG3 comprend un bandeau et trois zones d'intérêt P15, P16 et P17. Le bandeau comporte les icones de commande P1, P2 décrites précédemment, les zones d'intérêt P5, P6 décrites précédemment, permettant de répondre par oui ou non, une icône P18 permettant d'afficher la page d'accueil PG2. La zone d'intérêt P15 représente une échelle graduée de 1 à 10, dans laquelle le patient peut désigner un point pour exprimer un degré de douleur ressentie entre 1 et 10. La sélection d'un tel point déclenche la diffusion orale de la valeur du point désigné. La zone d'intérêt P16 donne accès à une page de communication permettant au patient d'indiquer l'emplacement de la région douloureuse. La zone d'intérêt P17 donne accès à une page permettant au patient de communiquer sur la manière dont il perçoit cette douleur.

La f igure 10 représente un exemple de page de communication PG4 sur l'emplacement de la région douloureuse, cette page étant affichée à la suite de la désignation de la zone d'intérêt P16 (f igure 9). La page PG4 comprend un bandeau, une fenêtre F1 présentant la face avant FS et la face arrière BS d'un corps humain, une fenêtre F2 d'affichage de texte et la zone d'intérêt P17. Le bandeau comprend celui de la page PG3 et une icône P19 permettant d'afficher à nouveau la page d'écran précédente PG3. Les faces avant FS et arrière BS permettent au patient de désigner la région douloureuse de leur corps. A la suite de la sélection d'une région du corps, cette région peut être indiquée sous la forme d'un texte diffusé oralement et/ou affiché dans la fenêtre F2. Les faces avant et arrière FS, BS présentées peuvent être adaptées au sexe et/ou à l'âge du patient.

La f igure 11 représente un exemple de page de communication PG5 sur la manière dont la douleur est ressentie, cette page étant affichée à la suite de la désignation de la zone d'intérêt P17 dans la page PG3 ou PG4. La page PG5 comprend un bandeau, la zone d'intérêt P16 de la page PG3, et onze zones d'intérêt P20 à P29. Le bandeau est identique au bandeau de la page PG4. Les zones d'intérêts P20, P21 permettent d'indiquer que la douleur est ressentie en continu et d'une manière discontinue, respectivement. Les zones d'intérêt P22, P23 permettent d'indiquer que la douleur apparaît d'une manière brutale, et progressive, respectivement. Les zones d'intérêt P24, P25, P26, P27, P28 permettent d'indiquer des sensations de démangeaison, de brûlure, de piqûre, d'engourdissement, et de crampe, respectivement. La zone d'intérêt P29 permet d'indiquer que la région désignée est insensible.

Le patient peut ainsi décrire précisément la douleur ressentie en procédant à six sélections de zones d'intérêt dans les pages PG2, PG3, PG4, PG5.

La f igure 12 représente un exemple de page de communication PG6 permettant au patient d'indiquer une sensation, cette page étant affichée à la suite de la désignation de la zone d'intérêt P12 de la page PG2. La page PG6 comprend un bandeau et huit zones d'intérêt P9 et P30 à P36, dont la zone d'intérêt P9 décrite précédemment (f igure 7). Le bandeau est identique à celui de la page PG3. Les zones d'intérêt P30, P34 permettent au patient d'indiquer qu'il a soif et faim, respectivement. Les zones d'intérêt P31, P35 permettent au patient d'indiquer qu'il a trop chaud et froid, respectivement. Les zones d'intérêt P32, P33 permettent au patient d'indiquer respectivement qu'il ressent une sensation d'étouffement, et qu'il a sommeil. La zone d'intérêt P36 permet au patient d'indiquer se sent angoissé ou dépressif.

La f igure 13 représente un exemple de page de communication PG7 permettant au patient d'exprimer un besoin concernant son environnement. La page PG7 est affichée à la suite de la désignation de la zone d'intérêt P11 de la page PG2. Cette page comprend un bandeau et six zones d'intérêt P40 à P45. Le bandeau est identique à celui de la page PG3. La zone d'intérêt P40 permet au patient d'indiquer qu'il souhaite changer de position sur son lit ou changer l'inclinaison du lit. Les zones d'intérêt P41, P44 permettent de demander respectivement qu'on allume ou éteigne la lumière, et qu'on ouvre ou ferme le volet de la fenêtre. La zone d'intérêt P42 permet au patient d'indiquer qu'il souhaite changer la température de consigne du chauffage ou du climatiseur de la chambre. La zone d'intérêt P43 permet au patient d'indiquer qu'il souhaite regarder la télévision. La zone d'intérêt P45 permet au patient d'indiquer qu'il éprouve une gêne en raison du bruit ambiant.

La f igure 14 représente un exemple de page d'accueil PG10 affichée par le module d'interface complexe CINT. La page d'accueil PG10 comprend un bandeau supérieur et huit zones d'intérêt comprenant les zones d'intérêt P4, P7, P10, P11, P12 et P13 de la page d'accueil PG2, et des zones d'intérêts P50, P51. Le bandeau supérieur affiche la date et l'heure courante DT, les trois pictogrammes P1, P2, P3 et les zones d'intérêt P5, P6 permettant au patient de répondre "OUI" et "NON", respectivement à une question qui lui est posée vocalement. Les zones d'intérêt P4, P7, P10, P12 et P13 donnent accès aux mêmes pages que celles de la page d'accueil PG2 du module d'interface simple SINT. La zone d'intérêt P50 donne accès à une page de commande permettant de sélectionner des systèmes de communication externes, comme le réseau Internet IN, le téléphone mobile du patient MP, et à une bibliothèque de documents multimédia, tels que des livres ou des films. La connexion au téléphone du patient peut être réalisée de manière à donner accès aux fonctionnalités du téléphone. La zone d'intérêt P51 donne accès à un clavier. La zone d'intérêt P11 permet d'afficher la page PG7 pour sélectionner un dispositif externe à commander présentés dans les zones d'intérêt P40 à P44 (lit, éclairage, volets, climatisation, ...). La sélection d'un de ces dispositifs externes dans la page PG7 active le module ECT permettant de commander directement ces dispositifs.

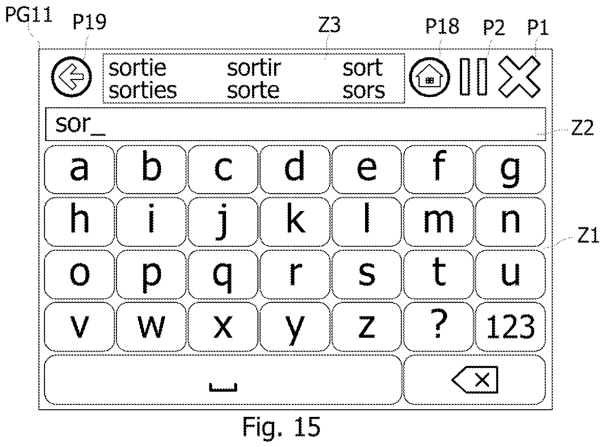

Le bandeau de chacune des pages affichées depuis la page d'accueil PG10 peut comporter la zone d'intérêt P51 donnant accès au clavier (Figure 15).

La f igure 15 représente un exemple de page PG11 présentant un clavier Z1, cette page étant affichée à la suite de la sélection de la zone d'intérêt ou icône P51. La page d'écran PG11 comprend également un bandeau présentant les icones de commande P1, P2, P18 et P19 déjà décrites, ainsi que des zones d'affichage Z2, Z3. La zone d'affichage Z2 présente les caractères sélectionnés à l'aide du clavier ou des mots sélectionnés dans la zone d'affichage Z3. La zone d'affichage Z3 présente des suggestions de mots désignables, sélectionnées par le dispositif de communication en fonction des caractères affichés dans la zone d'affichage Z2 du mot en cours de saisie. En désignant la zone Z2, le mot ou la phrase affichée dans cette zone peut être diffusée vocalement.

La page d'accueil affichée par le module d'interface complexe CINT donne ainsi accès à des moyens de communication étendus.

Selon un mode de réalisation, les différentes interfaces de communication peuvent être présentées de différentes manières, par exemple avec une couleur de fond distincte, pour pouvoir visualiser sur chaque page affichée à quelle interface de communication la page appartient.

Il apparaîtra clairement à l'homme de l'art que la présente invention est susceptible de diverses variantes de réalisation et diverses applications. En particulier, l'invention n'est pas limitée à la prévision de trois modules d'interface de communication ou trois interfaces de communications. En effet, il peut être prévu deux ou plus de trois interfaces de communication distinctes pour s'adapter aux capacités des patients à utiliser une telle interface.

Les tests cognitifs décrits précédemment ne sont que des exemples et d'autres formes de tests peuvent être prévues pour déterminer la capacité du patient à comprendre des consignes fournies oralement et ce qui est affiché à l'écran, et à désigner des zones d'intérêt sur un écran d'ordinateur.

L'organe de commande utilisé par le patient pour désigner des zones d'intérêt n'est pas nécessairement un dispositif d'oculométrie ou de suivi du regard, et le patient à qui s'adresse le dispositif de communication n'est pas nécessairement incapable de bouger une main. L'organe de commande peut donc être n'importe quel dispositif susceptible de permettre au patient de désigner une zone d'une page affichée sur un écran.

Dans certaines situations, le support 5 peut être omis, notamment lorsque le patient peut tenir une tablette entre ses mains.

## Revendications

1. Procédé de communication entre un patient incapacité n'ayant plus l'usage de la parole et un soignant ou un proche, comprenant des étapes exécutées par un ordinateur, consistant à :
équiper le patient d'un organe de commande (3) et d'un écran d'affichage (2) connectés à l'ordinateur,
exécuter une séquence de tests cognitifs comprenant les étapes suivantes :
(a) afficher une page comportant plusieurs images (I1-I8, I10-I13) à l'écran d'affichage (2),
(b) émettre une consigne vocale en relation avec les images de la page affichée,
(c) acquérir une réponse du patient en relation avec la page affichée, à l'aide de l'organe de commande (3), et
(d) mettre à jour un score de compréhension (SCR) en fonction de la réponse du patient, la réponse du patient étant un signal de sélection d'une zone de l'écran d'affichage ou des mouvements oculaires détectés à l'aide d'un dispositif d'oculométrie,
sélectionner une interface de communication en fonction du score de compréhension, parmi plusieurs interfaces de communication (EINT, SINT, CINT) mises en œuvre par l'ordinateur et ayant des complexités respectives distinctes, chaque interface de communication comprenant au moins une page d'accueil (PG1, PG2, PG10) affichable à l'écran d'affichage et comportant des zones d'intérêt sélectionnables (P1-P13, P50, P51) à l'aide de l'organe de commande, les zones d'intérêt correspondant à des messages que le patient est susceptible de communiquer au soignant ou au proche,
activer l'interface de communication sélectionnée et afficher à l'écran d'affichage la page d'accueil de l'interface de communication sélectionnée,
détecter la désignation à l'aide de l'organe de commande d'une zone d'intérêt parmi les zones d'intérêt dans une page (PG1-PG7, PG10, PG11) affichée à l'écran d'affichage, et
commander l'émission d'un message vocal destiné au soignant ou au proche, correspondant à la zone d'intérêt désignée.

2. Procédé selon la revendication 1, dans lequel les interfaces de communication (EINT, SINT, CINT) comprennent :
une première interface de communication (EINT) comprenant uniquement une page d'accueil (PG1) donnant accès à au plus dix commandes, et/ou
une seconde interface de communication (SINT) donnant accès à des commandes à partir de zones d'intérêt réparties sur la page d'accueil (PG2) et plusieurs autres pages (PG3-PG7), les commandes de la seconde interface de communication étant activables en au plus six sélections à l'aide de l'organe de commande (3), et/ou
une troisième interface de communication (CINT) donnant accès à des commandes réparties sur un nombre illimité de pages.

3. Procédé selon la revendication 1, comprenant l'incrémentation par l'ordinateur du score (SCR) lorsque le patient a maintenu son regard sur l'une des images (I1-I8, I10-I13) affichées, correspondant à la consigne vocale.

4. Procédé selon l'une des revendications 1 à 3, dans lequel les tests cognitifs comprennent des tests de compréhension de mots, des tests de compréhension de phrases simples et des tests de compréhension de phrases complexes.

5. Procédé selon la des revendication 4, dans lequel le score de compréhension (SCR) comptabilise un nombre de réponses correctes du patient en relation avec les tests de compréhension de mots, un nombre de réponses correctes du patient en relation avec les tests de compréhension de phrases simples et un nombre total de réponses correctes du patient pour la séquence de tests cognitifs.

6. Procédé selon la des revendication 5, comprenant, à la suite de l'exécution de la séquence de tests, des étapes exécutées par l'ordinateur, consistant à :
si le score de compréhension (SCR) indique que le nombre total de réponses correctes est supérieur à une première valeur de seuil, sélectionner une première interface de communication (CINT) donnant accès à des commandes réparties sur un nombre de pages illimité de pages,
sinon si le score de compréhension indique que le nombre de réponses correctes en relation avec les tests de compréhension de phrases simples est supérieur à une seconde valeur de seuil, sélectionner une seconde interface de communication (SINT) donnant accès à des commandes réparties sur plusieurs pages et activables en au plus six sélections à l'aide de l'organe de commande, et
sinon si le score de compréhension indique que le nombre de réponses correctes en relation avec les tests de compréhension de mots simples est supérieur à une troisième valeur de seuil, sélectionner une troisième interface de communication (EINT) donnant accès à au plus dix commandes,
sinon se mettre en attente de l'exécution d'une nouvelle séquence de test.

7. Procédé selon l'une des revendications 1 à 6, comprenant des étapes exécutées par l'ordinateur, consistant à :
sélectionner une séquence de tests cognitifs à exécuter pour déterminer un score de compréhension du patient, parmi plusieurs séquences de tests cognitifs, ou
sélectionner un nombre de planches dans des groupes de planches de même niveau de difficulté pour constituer une séquence de tests cognitifs à exécuter pour déterminer un score de compréhension du patient.

8. Procédé selon la revendication 7, dans lequel la sélection d'une séquence de tests cognitifs à exécuter est effectuée en excluant une ou deux dernières séquences de tests cognitifs précédemment sélectionnées.

9. Procédé selon l'une des revendications 1 à 8, comprenant des étapes exécutées par l'ordinateur, consistant à :
détecter au moyen d'un dispositif d'oculométrie (3) que le patient a maintenu son regard sur une première zone d'intérêt (P1-P13) dans une page (PG1-PG7, PG10, PG11) affichée à l'écran pendant une durée supérieure à une valeur de seuil temporel, et
commander l'émission d'un message vocal correspondant à la première zone d'intérêt, ou afficher une nouvelle page en relation avec la première zone d'intérêt.

10. Procédé selon l'une des revendications 1 à 9, comprenant des étapes exécutées par l'ordinateur, consistant à :
détecter par un dispositif d'oculométrie (3), une position de l'écran d'affichage (2) fixée par le patient,
afficher une icône (M1) à la position détectée,
déterminer que la position détectée est fixe,
lorsque la position détectée est fixe, animer l'icône pour indiquer un temps restant à fixer la position fixe, et
activer une commande correspondant à la position fixe détectée, lorsque le temps écoulé depuis que la position détectée est fixe, dépasse une valeur de seuil de temps écoulé.

11. Procédé selon la revendication 10, dans lequel la valeur de seuil de temps écoulé est fixée en fonction de l'interface de communication sélectionnée (EINT, SINT, CINT), et/ou en fonction du score de compréhension (SCR) obtenu lors de la dernière séquence de tests cognitifs exécutée.

12. Procédé selon l'une des revendications 1 à 11, dans lequel la troisième interface de communication (CINT) donne accès à un module de commande (ECT) permettant de commander directement des dispositifs externes (ENVC, MEDC) situés dans l'environnement proche du patient.

13. Dispositif de communication comprenant un ordinateur (PC), un écran d'affichage (2) et un organe de commande (3), l'ordinateur étant configuré pour mettre en œuvre le procédé selon l'une des revendications 1 à 12.

14. Dispositif selon la revendication 13, dans lequel l'organe de commande comprend un dispositif d'oculométrie (3) fournissant à l'ordinateur (PC) des positions successives du regard du patient sur l'écran (2), l'ordinateur étant configuré pour activer une commande associée à une zone d'intérêt (P1-P13) affichée à l'écran, lorsque la position du regard du patient fournie par le dispositif d'oculométrie est maintenue dans la zone d'intérêt pendant une durée supérieure à une valeur de seuil temporel.

## Patentansprüche

1. Verfahren zur Kommunikation zwischen einem behinderten Patienten, der sein Sprachvermögen nicht mehr verwenden kann, und einer Pflegekraft oder einem Angehörigen, umfassend durch einen Computer ausgeführte Schritte, bestehend aus:
Ausstatten des Patienten mit einer Befehlseinrichtung (3) und einem Anzeigebildschirm (2), die mit dem Computer verbunden sind,
Ausführen einer Sequenz von kognitiven Tests, umfassend die folgenden Schritte:
(a) Anzeigen einer Seite mit mehreren Bildern (I1-I8, I10-I13) auf dem Anzeigebildschirm (2),
(b) Ausgeben einer Sprachanweisung in Bezug auf die Bilder der angezeigten Seite,
(c) Erfassen einer Antwort des Patienten in Bezug auf die angezeigte Seite mittels der Befehlseinrichtung (3) und
(d) Aktualisieren einer Verständnisbewertung (SCR) in Abhängigkeit von der Antwort des Patienten, wobei die Antwort des Patienten ein Auswahlsignal eines Bereichs des Anzeigebildschirms ist oder Augenbewegungen sind, die mit Hilfe einer Okulometrievorrichtung erkannt werden,
Auswählen einer Kommunikationsschnittstelle in Abhängigkeit von der Verständnisbewertung aus mehreren Kommunikationsschnittstellen (EINT, SINT, CINT), die durch den Computer implementiert sind, und die jeweils verschiedene Komplexitäten aufweisen, jede Kommunikationsschnittstelle umfassend mindestens eine Startseite (PG1, PG2, PG10), die auf dem Anzeigebildschirm anzeigbar ist, und umfassend mittels der Befehlseinrichtung auswählbare Interessensbereiche (P1-P13, P50, P51), wobei die Interessensbereiche Nachrichten entsprechen, die der Patient an die Pflegekraft oder den Angehörigen zu kommunizieren imstande ist,
Aktivieren der ausgewählten Kommunikationsschnittstelle und Anzeigen auf dem Anzeigebildschirm der Startseite der ausgewählten Kommunikationsschnittstelle,
Erkennen der Bezeichnung mittels der Befehlseinrichtung eines Interessensbereichs unter den Interessensbereichen auf einer Seite (PG1-PG7, PG10, PG11), die auf dem Anzeigebildschirm angezeigt wird, und
Steuern der Aussendung einer Sprachnachricht, die für die Pflegekraft oder den Angehörigen vorgesehen ist, die dem bezeichneten Interessensbereich entspricht.

2. Verfahren nach Anspruch 1, wobei die Kommunikationsschnittstellen (EINT, SINT, CINT) umfassen:
eine erste Kommunikationsschnittstelle (EINT), umfassend ausschließlich eine Startseite (PG1), die Zugang zu höchstens zehn Befehlen gewährt, und/oder
eine zweite Kommunikationsschnittstelle (SINT), die Zugang zu Befehlen aus Interessensbereichen gewährt, die auf der Startseite (PG2) und mehreren anderen Seiten (PG3-PG7) verteilt sind, wobei die Befehle der zweiten Kommunikationsschnittstelle in höchstens sechs Auswahlen mittels der Befehlseinrichtung (3) aktivierbar sind, und/oder
eine dritte Kommunikationsschnittstelle (CINT), die Zugang zu Befehlen gewährt, die auf eine unbegrenzte Anzahl von Seiten verteilt sind.

3. Verfahren nach Anspruch 1, umfassend die Inkrementierung, durch den Computer, der Bewertung (SCR), wenn der Patient seinen Blick auf einem der angezeigten Bilder (I1-I8, I10-I13) gehalten hat, das der Sprachanweisung entspricht.

4. Verfahren nach einem der Ansprüche 1 bis 3, wobei die kognitiven Tests Verständnistests von Wörtern, Verständnistests einfacher Sätze und Verständnistests komplexer Sätze umfassen.

5. Verfahren nach Anspruch 4, wobei die Verständnisbewertung (SCR) eine Anzahl von korrekten Antworten des Patienten in Bezug auf die Verständnistests von Wörtern, eine Anzahl von korrekten Antworten des Patienten in Bezug auf die Verständnistests einfacher Sätze und eine Gesamtanzahl von korrekten Antworten des Patienten für die Sequenz kognitiver Tests zahlenmäßig ausdrückt.

6. Verfahren nach Anspruch 5, umfassend, im Anschluss an die Ausführung der Sequenz von Tests, durch den Computer ausgeführte Schritte, bestehend aus:
falls die Verständnisbewertung (SCR) angibt, dass die Gesamtzahl der korrekten Antworten größer als ein erster Schwellenwert ist, Auswählen einer ersten Kommunikationsschnittstelle (CINT), die Zugang zu Befehlen gewährt, die auf eine unbegrenzte Anzahl von Seiten verteilt sind,
andernfalls, falls die Verständnisbewertung angibt, dass die Anzahl von korrekten Antworten in Bezug auf die Verständnistests einfacher Sätze größer als ein zweiter Schwellenwert ist, Auswählen einer zweiten Kommunikationsschnittstelle (SINT), die Zugang zu Befehlen gewährt, die auf mehrere Seiten verteilt und in höchstens sechs Auswahlen mittels der Befehlseinrichtung aktivierbar sind, und
andernfalls, falls die Verständnisbewertung angibt, dass die Anzahl von korrekten Antworten in Bezug auf die Verständnistests einfacher Wörter größer als ein dritter Schwellenwert ist, Auswählen einer dritten Kommunikationsschnittstelle (EINT), die Zugang zu höchstens zehn Befehlen gewährt,
andernfalls in den Wartezustand für die Ausführung einer neuen Sequenz von Tests gehen.

7. Verfahren nach einem der Ansprüche 1 bis 6, umfassend die durch den Computer ausgeführten Schritte, bestehend aus:
Auswählen einer Sequenz kognitiver Tests zur Ausführung zum Bestimmen einer Verständnisbewertung des Patienten aus mehreren Sequenzen kognitiver Tests oder
Auswählen einer Anzahl von Tafeln in Gruppen von Tafeln von gleichem Schwierigkeitsgrad, zum Erstellen eine Sequenz kognitiver Tests, die zum Bestimmen einer Verständnisbewertung des Patienten auszuführen ist.

8. Verfahren nach Anspruch 7, wobei die Auswahl einer auszuführenden Sequenz kognitiver Tests durch Ausschluss einer oder zweier zuletzt zuvor ausgewählter Sequenzen kognitiver Tests erfolgt.

9. Verfahren nach einem der Ansprüche 1 bis 8, umfassend die durch den Computer ausgeführten Schritte bestehend aus:
Erkennen, mittels einer Okulometrievorrichtung (3), dass der Patient seinen Blick auf einen ersten Interessenbereich (P1-P13) auf einer Seite (PG1-PG7, PG10, PG11), die auf dem Bildschirm angezeigt wird, für eine Dauer, die größer als ein zeitlicher Schwellenwert ist, gehalten hat und
Befehlen des Aussendens einer Sprachnachricht, die dem ersten Interessensbereich entspricht, oder Anzeigen einer neuen Seite in Bezug auf den ersten Interessensbereich.

10. Verfahren nach einem der Ansprüche 1 bis 9, umfassend die durch den Computer ausgeführten Schritte bestehend aus:
Erkennen, durch eine Okulometrievorrichtung (3), eine durch den Patienten fixierte Position des Anzeigebildschirms (2),
Anzeigen eines Symbol (M1) an der erkannten Position,
Bestimmen, dass die erkannte Position fixiert ist,
wenn die erkannte Position fixiert ist, Animieren des Symbols zum Angeben einer verbleibenden Zeit zum Fixieren der fixierten Position und
Aktivieren eines Befehls, der der erkannten fixierten Position entspricht, wenn die verstrichene Zeit, seitdem die erkannte Position fixiert ist, einen Schwellenwert an verstrichener Zeit überschreitet.

11. Verfahren nach Anspruch 10, wobei der Schwellenwert für die verstrichene Zeit in Abhängigkeit von der ausgewählten Kommunikationsschnittstelle (EINT, SINT, CINT) und/oder in Abhängigkeit von der Verständnisbewertung (SCR) fixiert wird, die bei der letzten ausgeführten Sequenz kognitiver Tests erhalten wurde.

12. Verfahren nach einem der Ansprüche 1 bis 11, wobei die dritte Kommunikationsschnittstelle (CINT) Zugang zu einem Steuermodul (ECT) gewährt, das ein direktes Befehlssteuern von externen Vorrichtungen (ENVC, MEDC) ermöglicht, die in der unmittelbaren Umgebung des Patienten gelegen sind.

13. Kommunikationsvorrichtung, umfassend einen Computer (PC), einen Anzeigebildschirm (2) und eine Befehlseinrichtung (3), wobei der Computer zum Implementieren des Verfahrens nach einem der Ansprüche 1 bis 12 konfiguriert ist.

14. Vorrichtung nach Anspruch 13, wobei die Befehlseinrichtung eine Okulometrievorrichtung (3) umfasst, die dem Computer (PC) aufeinanderfolgende Positionen des Blicks des Patienten auf dem Bildschirm (2) bereitstellt, wobei der Computer zum Aktivieren eines Befehls konfiguriert ist, der einer auf dem Bildschirm angezeigten Interessenszone (P1-P13) zugeordnet ist, wenn die Position des Blicks des Patienten, die durch die Okulometrievorrichtung bereitgestellt wird, in der Interessenszone für eine Dauer gehalten wird, die größer als ein zeitlicher Schwellenwert ist.

## Claims

1. A communication method between an incapacitated speechimpaired patient and a caregiver or a relative, comprising steps carried out by a computer, consisting in:
providing the patient with a control device (3) and a display screen (2) connected to the computer,
executing a sequence of cognitive tests comprising the following steps:
(a) displaying a page comprising several images (I1-I8, 110-113) on the display screen (2),
(b) issuing a voice command in relation to the images on the displayed page,
(c) acquiring a response from the patient in relation to the page displayed, using the control device (3), and
(d) updating the comprehension score (SCR) as a function of the patient's response, wherein the patient's response is a selection signal for an area of the display screen or eye movements detected using an eye-tracking device.
selecting a communication interface as a function of the comprehension score, from a plurality of communication interfaces (EINT, SINT, CINT) implemented by the computer and having distinct respective complexities, each communication interface including at least one home page (PG1, PG2, PG10) displayable on the display screen and including selectable areas of interest (P1-P13, P50, P51) using the control device, the areas of interest corresponding to messages that the patient may communicate to the caregiver or the relative,
activating the selected communication interface and displaying the home page of the selected communication interface,
detecting a designation by the control device of an area of interest among the areas of interest in a page (PG1-PG7, PG10, PG11) displayed on the display screen, and
controlling the emission of a voice message destined to the caregiver or the relative corresponding to the designed area of interest.

2. The method according to claim 1, wherein the communication interfaces (EINT, SINT, CINT) comprise:
a first communication interface (EINT) comprising only a home page (PG1) providing access to at most ten commands, and/or
a second communication interface (SINT) providing access to commands from areas of interest distributed over the home page (PG2) and several other pages (PG3-PG7), the commands of the second communication interface being available in at most six selections using the control device (3), and/or
a third communication interface (CINT) providing access to commands distributed over an unlimited number of pages.

3. The method according to claim 2, comprising the incrementation by the computer of the score (SCR) when the patient has maintained his/her gaze on one of the images (I1-I8, 110-113) displayed, corresponding to the vocal instruction.

4. The method according to one of claims 1 to 3, wherein the cognitive tests comprise word comprehension tests, simple sentence comprehension tests, and complex sentence comprehension tests.

5. The method according to claim 4, wherein the comprehension score (SCR) counts a number of correct responses in relation to word comprehension tests, a number of correct responses in relation to simple sentence comprehension tests, and a total number of correct responses for the cognitive test sequence.

6. The method according to claim 5, comprising, following the execution of the test sequence, steps executed by the computer, comprising:
if the comprehension score (SCR) indicates that the total number of correct answers is greater than a first threshold value, selecting a first communication interface (CINT) providing access to commands distributed over an unlimited number of pages,
otherwise, if the comprehension score indicates that the number of correct answers in relation to the simple sentence comprehension tests is greater than a second threshold value, selecting a second communication interface (SINT) providing access to commands distributed over several pages and reachable in no more than six selections using the control device, and
otherwise, if the comprehension score indicates that the number of correct answers in relation to the simple word comprehension tests is greater than a third threshold value, selecting a third communication interface (EINT) providing access to up to ten commands,
otherwise waiting for a new test sequence to be executed.

7. The method according to one of claims 1 to 6, comprising computer-executed steps of:
selecting a sequence of cognitive tests to determine a patient's comprehension score, from a plurality of sequences of cognitive tests, or
selecting a number of slides from groups of slides of a same difficulty level to form a sequence of cognitive tests to determine a patient comprehension score.

8. The method according to claim 7, wherein the selection of a sequence of cognitive tests is performed by excluding one or two most recent previously selected sequences of cognitive tests.

9. The method according to one of claims 1 to 8, comprising computer-executed steps of:
detecting by an eye-tracking device (3) that the patient has maintained his/her gaze on a first area of interest (P1-P13) in a page (PG1-PG7, PG10, PG11) displayed on the screen for a duration greater than a temporal threshold value, and
controlling the emission of a vocal message corresponding to the first area of interest, or displaying a new page related to the first area of interest.

10. The method according to one of claims 1 to 9, comprising computer-executed steps of:
detecting by an eye-tracking device (3) a position in the display screen (2) gazed at by the patient,
displaying an icon (M1) at the detected position,
determining that the detected position is static,
when the detected position is static, animating the icon to indicate a time remaining to gaze at the static position, and
activating a command corresponding to the detected static position when the elapsed time since the position is detected as static exceeds a time threshold value.

11. The method according to claim 10, wherein the time threshold value is set as a function of the selected communication interface (EINT, SINT, CINT), and/or as a function of the comprehension score (SCR) obtained during the last performed cognitive test sequence.

12. The method according to one of claims 1 to 11, wherein the third communication interface (CINT) provides access to a control module (ECT) enabling direct control of external devices (ENVC, MEDC) located in the patient's immediate environment.

13. A communication device comprising a computer (PC), a display screen (2), and a control device (3), wherein the computer is configured to implement the method according to one of claims 1 to 12.

14. The device according to claim 13, wherein the control device comprises an eye-tracking device (3) supplying the computer (PC) with successive positions of the patient's gaze on the screen (2), the computer being configured to activate a command associated with an area of interest (P1-P13) displayed on the screen, when the position of the patient's gaze supplied by the eye-tracking device is maintained in the area of interest for a duration greater than a time threshold value.
